Europäisches Patentamt

European Patent Office

Office européen des brevets

(11) Publication number: **0 512 867 A2**

# EUROPEAN PATENT APPLICATION

(21) Application number: **92304195.8**

(22) Date of filing: **11.05.92**

(51) Int. Cl.5: **G05B 19/18**, B23Q 15/12

(30) Priority: **10.05.91 GB 9110171**

(43) Date of publication of application:
**11.11.92 Bulletin 92/46**

(84) Designated Contracting States:
**DE FR GB IT**

(71) Applicant: **UNIVERSITY OF BRISTOL**
**Senate House Tyndall Avenue**
**Bristol BS8 1TH(GB)**

(72) Inventor: **Brett, Peter Nigel**
**"Pendragon"; 3 Manor Road**
**Saltford Bristol BS18 3 DL(GB)**
Inventor: **Khodabandehloo, Koorosh**
**45 Shadwell Road**
**Bishopston Bristol BS7 8EW(GB)**

(74) Representative: **Cheyne, John Robert**
**Alexander Mackenzie**
**HASELTINE LAKE & CO. 28 Southampton**
**Buildings Chancery Lane**
**London WC2A 1AT(GB)**

(54) **Removing material from a workpiece.**

(57) A tool in the form of a drill 20 is rotated by a motor 18 and advanced by a motor 8 acting through a lead screw 4. A transducer 30 monitors the feed force applied to the drill 20, and the current drawn by the motor 18, which corresponds to the torque applied to the drill 20, is also monitored. It has been found that the feed force decreases, and the torque increases, shortly before the drill 20 breaks through a workpiece, and so control means 56 responsive to feed force and torque can be adapted to arrest the feed movement shortly before breakthrough. This facility is particularly useful in surgical procedures.

*FIG.1*

This invention relates to equipment for removing material from a workpiece, and is particularly, although not exclusively, concerned with drilling operations.

In some drilling operations, it is necessary to stop drilling before the drill bit completely penetrates the workpiece. For example, in the surgical procedure known as stapedotomy a hole is drilled through the footplate of the stapes in the middle ear so that a prosthesis can be inserted. It is not possible for the surgeon to determine accurately the thickness of the bone, yet he must detect when the drill has broken through it so as to avoid drilling further into the inner ear with possibly dire consequences. There are many other examples, both surgical and non-surgical, of drilling or other machining operations in which drill or tool breakthrough must be avoided, or at least accurately detected, either at a surface of the workpiece, or at the interface between different strata of the workpiece.

According to the present invention there is provided equipment for removing material from a workpiece, the equipment comprising:

i) a rotary tool;

ii) rotary drive means for rotating the tool;

iii) feed means for displacing the tool towards and away from the workpiece;

iv) sensing means for providing output data representing the force and/or torque applied to the tool by the feed means and/or the rotary drive means; and

v) control means for controlling the feed means in response to the output data.

It has been found that, if the feed means is operated to maintain a constant rate of feed of the tool into the workpiece, the force applied to the tool will fall shortly before the tool breaks through the workpiece. Consequently, if the sensing means monitors the force applied to the tool, it is possible to arrest the advance of the tool shortly before breakthrough, or when the tool has only partly emerged from the workpiece.

It has also been found that, shortly before breakthrough, the torque applied to the tool to maintain its rotational speed increases significantly, and consequently, if the sensing means monitors the torque applied to the tool, the output data can also be used to control the feed means during a drilling or other material removal operation on the workpiece.

The control means may be adapted to maintain a constant feed rate, or alternatively a constant force or torque as monitored by the sensing means. The control means may be adapted to arrest the feed means when the force applied to the tool begins to fall, or when the rate of fall of the force exceeds a predetermined level. Alternatively, the control means may be adapted to arrest the feed of the tool when the torque applied to the tool crosses a predetermined threshold, or when the rate of change of the torque exceeds a predetermined level.

In a preferred embodiment, the rotary drive means and the tool are mounted on a carriage which is displaceable by the feed means on a stationary frame. The rotary drive means and the tool may be supported on the carriage by a resilient mounting with a single degree of freedom which enables them to move relatively to the carriage in the feed direction. The force sensing means may then comprise a transducer which is supported on the carriage and engaged by the rotary drive means (which may comprise an electric motor).

Where torque sensing means is provided, this means may comprise a torque transducer, for example circuitry for monitoring the current drawn by a d.c. electric motor constituting the rotary drive means.

For a better understanding of the present invention, and to show how it may be carried into effect, reference will now be made, by way of example, to the accompanying drawings, in which:

Figure 1 is a diagrammatic side view of surgical drilling equipment;

Figure 2 shows electrical circuitry of a motor of the equipment of Figure 1;

Figure 3 is a block diagram representing the control circuitry of the equipment;

Figure 4a and 4b show, respectively, the variation in feed force and motor torque when drilling through plastic using the equipment of Figure 1; and

Figures 5a and 5b show, respectively, the variation, in feed force and motor torque when drilling through bone using the equipment of Figure 1.

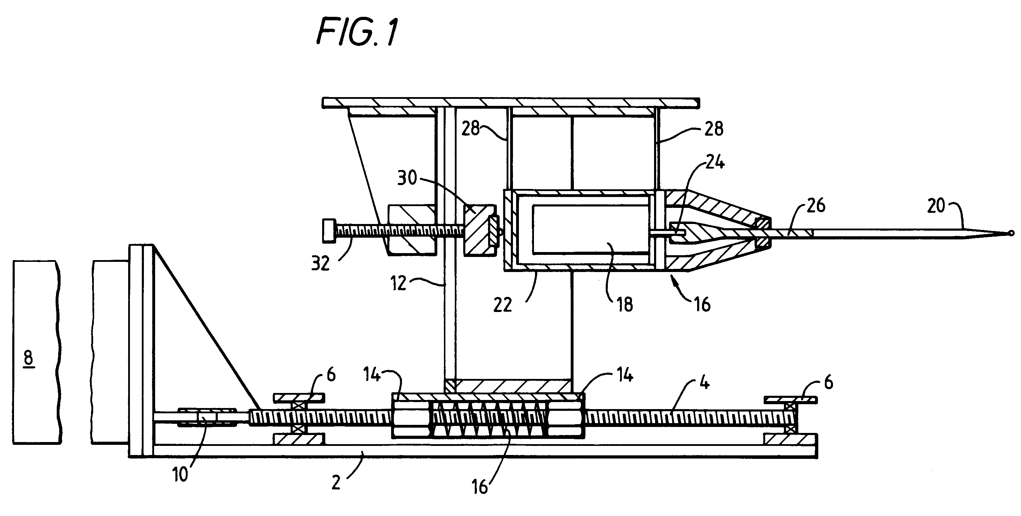

The equipment shown in Figure 1 is designed specifically for conducting a stapedotomy operation under robot control. However, the principles underlying the construction and operation of the equipment shown in Figure 1 can be applied to drilling equipment and other material removing equipment for both surgical and non surgical applications.

The equipment shown in Figure 1 comprises a base structure 2 on which a lead screw 4 is supported in bearings 6. The lead screw 4 is driven at one end by a feed motor 8 which includes reduction gearing and which is connected to the lead screw 4 by means of a flexible coupling 10.

A carriage 12 is supported for movement along the base structure 2 on two parallel rails (not shown). The carriage 12 is provided with two spaced nuts 14 through which passes the lead screw 4. The nuts 14 are biassed apart from each other by a spring 16, to eliminate backlash.

The carriage supports a surgical drill 16, which comprises a d.c. electric motor 18 and a drill bit 20. The motor 18 is accommodated in a housing 22, and its output shaft 24 drives a drill bit holder 26 in which the drill bit 20 is inserted.

The motor housing 22 is supported on the carriage 12 by two brass straps 28. These straps permit resilient displacement of the drill 16 in a direction parallel to that of the lead screw 4, but support the drill 16 relatively rigidly against displacement in directions perpendicular to that of the lead screw 4. The motor housing 22 abuts a force transducer 30 which is engaged by an adjustment screw 32.

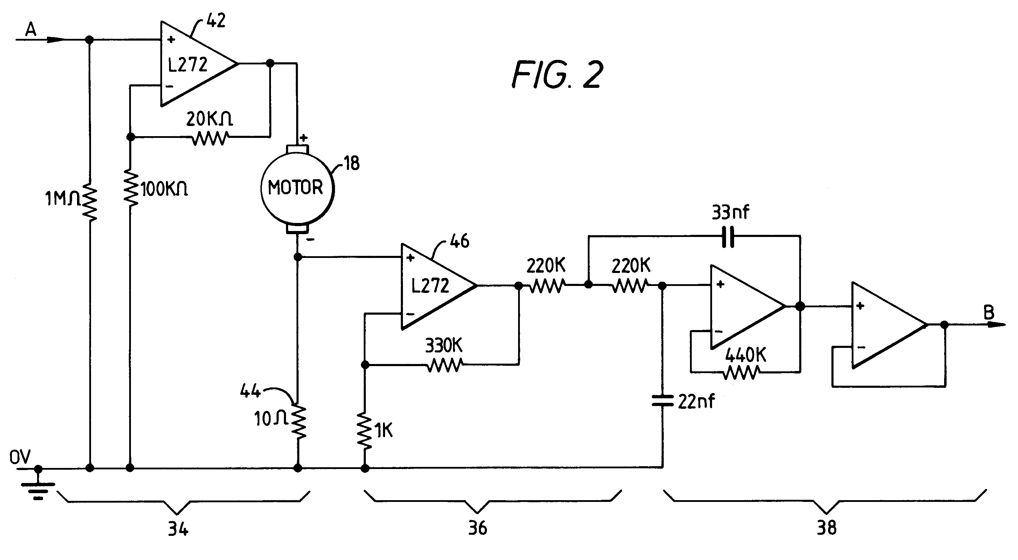

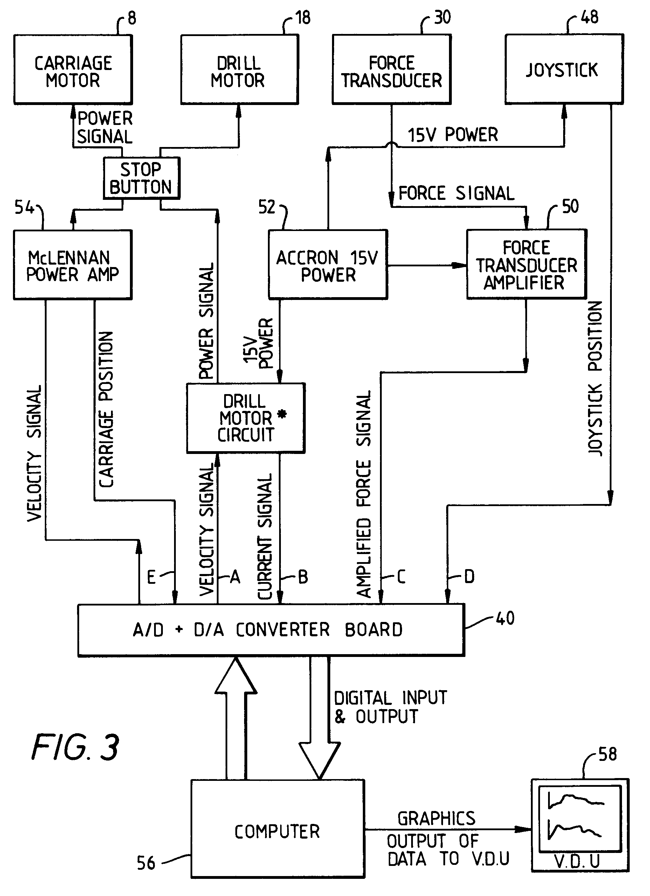

Figure 2 shows typical operating and monitoring circuitry for the motor 18. This circuitry is in three stages, of which the first stage, stage 34, is a power stage, stage 36 is a signal generating stage, and stage 38 is a filtering stage. In the power stage 34, an input signal A derived from an analog/digital and digital/analog converter board 40 (see Figure 3) is amplified by a factor of 1.2 by a power amplifier 42 to provide the required voltage for driving the motor 18 at a desired speed. The signal A is in the range 0 to 10 volts, giving a power signal of 0 to 12 volts after amplification.

The signal generating stage 36 detects the voltage across a 10Ω resistor 44. This voltage is amplified by a power amplifier 46 to give a signal representing the current drawn by the motor 18. This signal is filtered in the filtering stage 38 which is a second order low pass 20hz filter. The output signal B from the filtering stage 38 which is representative of the torque applied to the drill bit 20 by the motor 18, is supplied to the converter board 40.

Figure 3 is a block diagram representing the equipment of Figure 1 and its control circuitry.

As can be seen from Figure 3, the converter board 40 receives not only the signal B representing the current drawn from the motor 18, but also a force signal C generated by the transducer 30, a joystick position signal D generated by a joystick 48 and a carriage position signal E representing the position of the carriage 12 along the base 2. The joystick 48, a force transducer amplifier 50 and the drill motor circuit shown in Figure 2 receive power from a 15 volt power supply 52. The force transducer amplifier 50 amplifies the signal from the force transducer 30 to produce the signal C.

The carriage motor 8 is driven by a power amplifier 54, and a position transducer in the form of a rotary potentiometer (not shown) is provided to monitor rotation of the lead screw 4, so as to provide a signal representing the linear position of the carriage 12.

A computer 56 provides overall control of the equipment via a 2-way connection with the converter board 40. A VDU 58 connected to the computer provides graphical output of data.

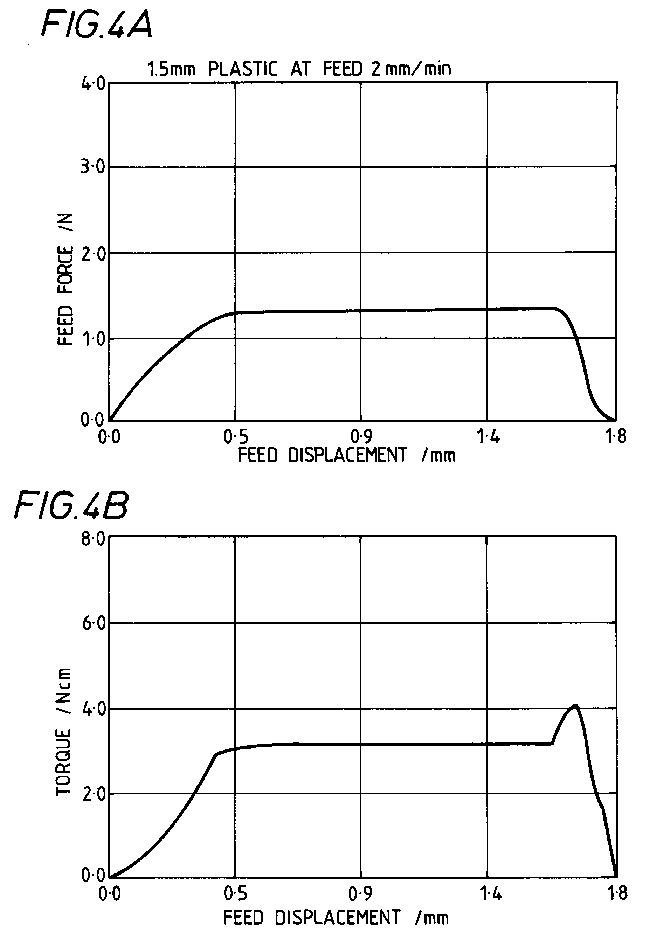

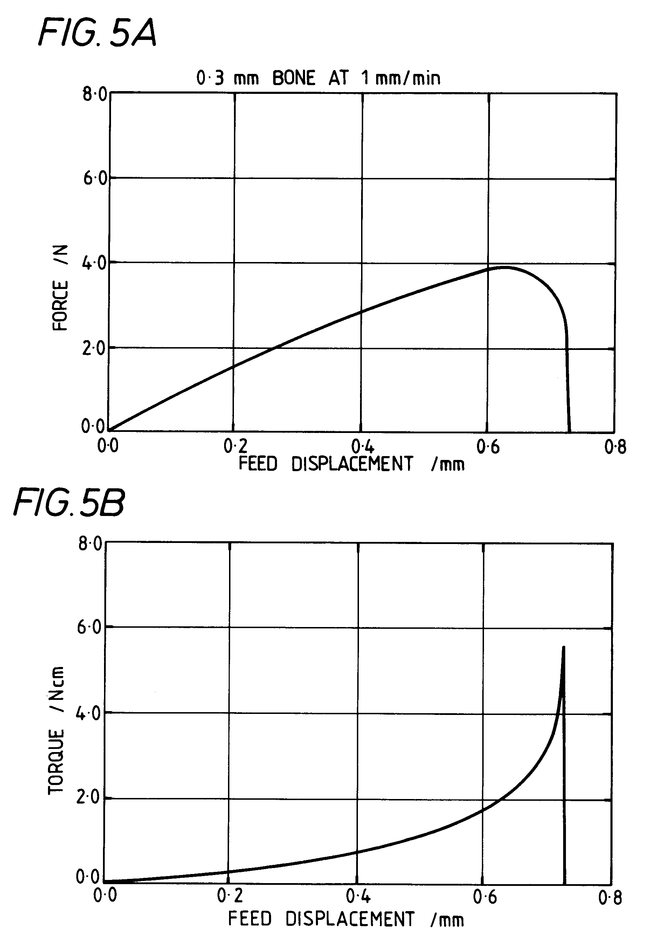

Figures 4A, 4B and 5A, 5B represent the force and torque applied to the drill bit 20 when drilling into different materials. Figure 4A shows the variation in feed force, as detected by the transducer 30, with feed displacement, generated from rotation of the lead screw 4, when drilling into a 1.5mm thick sheet of plastics material at a feed speed of 2mm per minute. It will be appreciated from the graph that the feed force increases as the drill bit 20 enters the material, and then remains substantially constant until the drill bit is close to breakthrough. The force then falls off rapidly as breakthrough occurs. The tests were performed using a spherical burr drill having a radius of 0.3mm, and consequently it will be appreciated that the full diameter of the drill had penetrated the material only after the drill had moved 0.3mm from the initial contact point. Similarly, full breakthrough occurred 0.3mm after the leading point of the drill first emerged from the material.

As shown in Figure 4B, the torque applied by the motor 18 increased as the drill entered the material and then remained constant as the drill bit progressed through the material. Just before breakthrough, the torque rose to define a pronounced peak before falling away to zero.

Either the reduction in feed force, or the increasing torque, or both, can be used to identify imminent breakthrough.

Similar results, at least at the point approaching breakthrough, are obtained when drilling bone, as shown in Figures 5A and 5B. The bone sample used in the test represented in Figures 5A and 5B was 0.3mm thick, that is the same as the radius of the burr drill. Consequently, the force and torque increase continuously with displacement, without reaching a plateau, as in Figures 4A and 4B, although there is a rapid increase in torque as breakthrough approaches.

Different material will provide force and torque curves having different characteristic features. However, for each material, there will be one or more characteristic features which can be used to cause the equipment to react appropriately. Matching techniques can be used so that the material being drilled can be recognized, and so that appropriate criteria can be established for, for example, termination of the drilling operation.

By appropriate programming of the computer 56, different modes of operation can be achieved. For example, the drill can be advanced until it contacts the material to be drilled, at which point the advance can be arrested in response to the reaction force exerted on the drill 20 when contact is made. Alternatively, the drill can be retreated by a predetermined amount, for example 0.3mm, after contact is made, to enable the operator to confirm that contact has been made at the correct position.

Another mode of operation is for the drill to be advanced until contact is made with the material after which the drill is further advanced to form a hole of a required, predetermined, depth, following which the drill is automatically retracted.

As mentioned above, another mode of operation is for a drilling operation to continue until breakthrough is detected. This can be achieved by, for example, drilling until the force detected by the transducer 30 falls to below 0.2 N. Of course, the threshold force can be adapted to suit particular circumstances or materials, and the degree of breakthrough which is required.

It is also possible for the drill to be moved by the motor 8 to a set position at any speed, or for the drill to be operated under the control of the joystick 48.

A useful feature of the equipment described above, particularly in surgical applications, is that, by monitoring bone contact and breakthrough, it is possible to gauge accurately the thickness of the bone being drilled. In the case of a stapedotomy operation, this enables the surgeon to select a prosthesis of an appropriate length for the thickness of the stapes footplate of the patient.

Although the present invention has been described mainly in connection with surgical operations, it will be appreciated that the principles underlying the invention can be applied to other forms of material removing operations. For example, the invention could be applied to the drilling of materials other than bone, for example wood, metal and plastics materials. As well as detecting drill breakthrough, the equipment could be adapted to detect a transition between two strata in a laminated material, such as melamine. Furthermore, the scope of the invention is not confined to the drilling of holes; it is also appropriate to, for example, the cutting of a slot using a circular cutter so as to cut a slot having a depth just short of the thickness of the material in which the slot is formed.

Additionally, although the examples given above relate to drilling operations in which the linear speed of the drill is constant, it is possible to program the computer 56 to provide a variable speed. For example, some materials will deflect under their own compliance, with the result that sudden breakthrough could occur when the deflected material springs back. To avoid this, the drilling operation could be controlled so that, as breakthrough approaches, the feed speed reduces, or even reverses, so that the final stages in the drilling operation are performed while the material being drilled returns to its unstressed state.

The characteristic changes in feed force and torque which take place as breakthrough or other transitional stages occur will vary with the nature of the material, its thickness, and the type and condition of the drill bit. By appropriate programming of the computer 56, it is possible to adapt the drilling operation to these factors or even to diagnose the factors themselves. For example, the computer could compare data generated during a drilling operation with data from previously performed operations stored in its memory, and provide an indication of the nature of material which is being drilled.

## Claims

1. Equipment for removing material from a workpiece, comprising:
   i) a rotary tool (20);
   ii) rotary drive means (18) for rotating the tool (20);
   iii) feed means (8,4,14) for displacing the tool (20) towards and away from the workpiece; characterized in that
   sensing means (30,36) is provided for providing output data representing the force and/or torque applied to the tool (20) by the feed means (8,4,14) and/or the rotary drive means (18), and control means (56) is provided for controlling the feed means (8,4,14) in response to the output data.

2. Equipment as claimed in claim 1, characterized in that the control means (56) is adapted to maintain a constant feed rate of the tool (20).

3. Equipment as claimed in claim 2, characterized in that the control means (56) is adapted to arrest the feed of the tool (20) when the feed force applied to the tool begins to fall.

4. Equipment as claimed in claim 2, characterized in that the control means (56) is adapted to arrest the feed of the tool (20) when the rate of fall of the feed force applied to the tool (20) exceeds a predetermined threshold.

5. Equipment as claimed in claim 2, characterized in that the control means (56) is adapted to arrest the feed of the tool (20) when torque applied to the tool (20) crosses a predetermined threshold.

6. Equipment as claimed in claim 2, characterized in that the control means (56) is adapted to arrest the feed of the tool (20) when the rate of charge of the torque applied to the tool (20) exceeds a predetermined level.

7. Equipment as claimed in any one of the preceding claims, characterized in that the rotary drive means (18) and the tool (20) are mounted on a carriage (12) which is displaceable by the feed means (8,4,14) relatively to a frame (2).

8. Equipment as claimed in claim 7, characterized in that the rotary drive means (18) and the tool (20) are supported on the carriage by a resilient mounting (28) which permits displacement of the rotary drive means (18) and the tool (20) relatively to the carriage (12) in the feed direction.

9. Equipment as claimed in claim 8, characterized in that the sensing means (30) comprises a transducer which is supported on the carriage (12) and engaged by the rotary drive means (18).

10. Equipment as claimed in any one of the preceding claims, characterized in that the sensing means (36) comprises circuitry for monitoring the current drawn by a d.c. electric motor constituting the rotary drive means (18).

FIG.1

FIG. 2

FIG. 3

*FIG.4A*

1.5mm PLASTIC AT FEED 2 mm/min

*FIG.4B*

# FIG. 5A

0·3 mm BONE AT 1 mm/min

FORCE /N vs FEED DISPLACEMENT /mm

# FIG. 5B

TORQUE /Ncm vs FEED DISPLACEMENT /mm